(19) Europäisches Patentamt
European Patent Office
Office européen des brevets

(11) EP 4 243 146 B1

(12) EUROPEAN PATENT SPECIFICATION

(45) Date of publication and mention of the grant of the patent:
**06.11.2024 Bulletin 2024/45**

(21) Application number: **21888469.0**

(22) Date of filing: **28.10.2021**

(51) International Patent Classification (IPC):
***H01M 10/0567*** *(2010.01)* ***H01M 10/0525*** *(2010.01)*
***C07D 497/10*** *(2006.01)* ***C07F 5/04*** *(2006.01)*
***C07F 9/6574*** *(2006.01)* ***H01M 10/42*** *(2006.01)*

(52) Cooperative Patent Classification (CPC):
**C07F 5/04; C07F 9/65744; H01M 10/0525; H01M 10/0567; H01M 10/42;** C07D 497/10; Y02E 60/10

(86) International application number:
**PCT/CN2021/126864**

(87) International publication number:
**WO 2022/095772 (12.05.2022 Gazette 2022/19)**

# (54) LITHIUM ION BATTERY NON-AQUEOUS ELECTROLYTE AND LITHIUM ION BATTERY

NICHTWÄSSRIGER ELEKTROLYT FÜR LITHIUM-IONEN-BATTERIE UND LITHIUM-IONEN-BATTERIE

ÉLECTROLYTE NON AQUEUX DE BATTERIE AU LITHIUM-ION ET BATTERIE AU LITHIUM-ION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.11.2020 CN 202011209851**

(43) Date of publication of application:
**13.09.2023 Bulletin 2023/37**

(73) Proprietor: **Shenzhen Capchem Technology Co., Ltd**
**Shenzhen, Guangdong 518118 (CN)**

(72) Inventors:
- **CAO, Chaowei**
**Shenzhen, Guangdong 518118 (CN)**
- **WANG, Chi**
**Shenzhen, Guangdong 518118 (CN)**
- **CHEN, Qun**
**Shenzhen, Guangdong 518118 (CN)**
- **HU, Shiguang**
**Shenzhen, Guangdong 518118 (CN)**

(74) Representative: **Herrero & Asociados, S.L.**
**Edificio Aqua - Agustín de Foxá, 4-10**
**28036 Madrid (ES)**

(56) References cited:
**EP-A1- 2 851 990**
**CN-A- 104 466 246**
**CN-A- 106 252 710**
**CN-A- 109 627 256**
**CN-A- 109 950 621**
**CN-A- 110 444 804**
**CN-A- 111 048 831**
**US-A1- 2016 211 550**
**US-A1- 2016 359 196**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 243 146 B1

## Description

### Technical field

The application belongs to the technical field of new energy, in particular to a non-aqueous electrolyte for a lithium ion battery and a lithium ion battery.

### Background

In recent years, lithium-ion batteries have been widely used in various portable electronic communication devices because of their high working voltage, no memory effect, long life, wide working temperature range and high safety. Under the background of updating national industrial planning policy on new energy vehicles, lithium-ion batteries, as the power supply of new energy electric vehicles, will usher in a period of explosive growth in demand.

In non-aqueous electrolyte lithium-ion batteries, non-aqueous electrolyte, as a medium for transporting and conducting current between anode and cathode, is the key factor to determine the performance of the batteries. In the first charging process, the compounds of non-aqueous electrolyte first undergo a reduction and decomposition reaction on the surface of the negative electrode, resulting in compounds such as lithium alkoxy, $Li_2CO_3$, lithium sulfonate, etc., and a passivation film is formed on the surface of the negative electrode, which is called solid electrolyte interface film (SEI). A good passive SEI film has thermal stability and chemical stability, which not only prevents the electrolyte from further decomposing on the surface of the carbon negative electrode, but also allows lithium ions to freely enter and exit the electrode while preventing solvent molecules from passing through, thus inhibiting the damage of solvent molecules to the electrode. Therefore, the quality of SEI film formation determines the performance of lithium-ion batteries.

With the wider application of lithium-ion batteries in different fields, people have higher requirements for the performance of lithium-ion batteries in market. By adding different negative film-forming additives (such as vinylene carbonate, ethylene sulfite and 1,3- propane sultone) to the electrolyte, researchers are able to improve the quality of SEI film, thus improving the cycle life and reversible capacity of the battery. For example, in Japanese Patent Laid-Open No. 2000-123867, it is proposed that if vinylene carbonate is added to the electrolyte, it can take precedence over solvent molecules, and the reduction and decomposition reaction will occur on the surface of the negative electrode to generate polyalkoxylithium carbonate compound, and a passive film will be formed on the surface of the negative electrode to prevent the electrolyte from further decomposition on the electrode surface, thus improving the cycle performance of the battery performance. However, after adding vinylene carbonate, the battery is likely to produce gas during high-temperature storage, which leads to the swelling of the battery. Ethylene sulfite can also play a role in inhibiting the decrease of the initial capacity of the battery, increasing the initial discharge capacity, reducing the expansion of the battery after being stored at high temperature, and improving the charging and discharging performance and cycle times of the battery. However, the SEI film formed by such compounds at the interface of graphite negative electrode has poor stability. With the increase of cycle times, the interface resistance of the electrode gradually increases, and the electronic polarization phenomenon of the battery becomes more and more serious, which leads to the rapid decrease of the reversible capacity of the electrode. The high-voltage additive, represented by 1,3-propane sultone additive, makes the positive electrode active material contact with the electrolyte and inhibits the oxidative decomposition of the electrolyte under high voltage by preferentially generating oxidation reaction on the positive electrode surface and forming a dense passivation film on the positive electrode surface. However, at present, higher requirements are put forward for high-temperature storage and cycle performances of lithium-ion batteries in the market.

US2016211550A1 discloses a non-aqueous electrolyte for a lithium ion battery, comprising a spiro compound.

### Summary

In view of the above technical problems, the present application provides a non-aqueous electrolyte for a lithium ion battery and a lithium ion battery, aiming at improving the high-temperature cycle and high-temperature storage performances of high-voltage lithium ion batteries and inhibiting the gas expansion phenomenon.

In order to solve the above technical problems, in one aspect, an embodiment of the present application provides a non-aqueous electrolyte for a lithium ion battery, including a non-aqueous organic solvent, a lithium salt, and a spiro compound represented by Structural Formula 1,

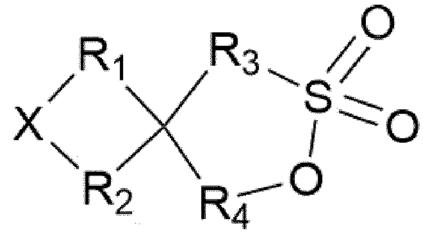

(Structural Formula 1)

wherein, X represents an oxygen atom, a carboxylate ester, a sulfite ester, a sulfate ester, a carbonate ester, a substituted or unsubstituted phosphate ester, and a substituted or unsubstituted borate ester; and

$R_1$, $R_2$, $R_3$ and $R_4$ are each independently selected from substituted or unsubstituted C1-C5 hydrocarbylene group.

Optionally, at least one of $R_1$, $R_2$, $R_3$ and $R_4$ is a substituted or unsubstituted C1-C5 alkylene group; a substituent of C1-C5 alkylene group includes one or more of a halogen-substituted or unsubstituted C1-C20 alkyl group, a halogen-substituted or unsubstituted C2-C20 alkenyl group, a halogen-substituted or unsubstituted C2-C20 alkynyl group, a halogen-substituted or unsubstituted C3-C20 cycloalkenyl group, a halogen-substituted or unsubstituted C3-C20 heterocyclic group, a halogen-substituted or unsubstituted C6-C40 aryl group, a halogen-substituted or unsubstituted C2-C40 heteroaryl group or a polar functional group with at least one heteroatom.

Optionally, at least one of $R_1$, $R_2$, $R_3$ and $R_4$ is a substituted or unsubstituted C1-C5 alkylene group; the substituent of C1-C5 alkylene group is selected from one or more of halogen, methyl, ethyl, propyl, isopropyl, butyl, tert-butyl, alkylene, alkynyl, phenyl, trimethylsilyl, cyano, trifluoromethyl, tetrafluoroethyl, naphthyl, tetrafluorophenyl, pyrrolyl or pyridyl.

Optionally, the spiro compound represented by Structural Formula 1 includes at least one of Formulas 1 to 7,

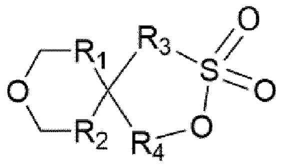

Formula 1

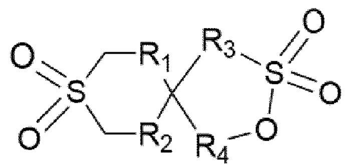

Formula 2

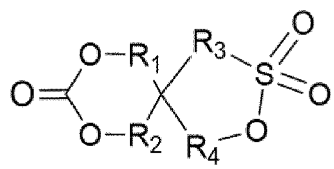

Formula 3

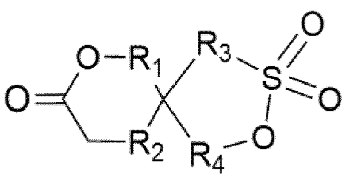

Formula 4

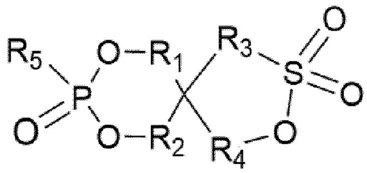

Formula 5

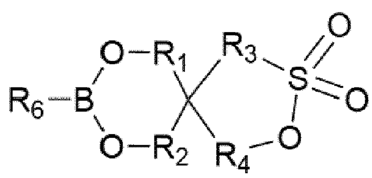

Formula 6

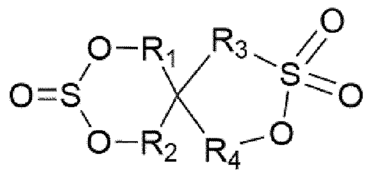

Formula 7

wherein $R_5$ and $R_6$ are each independently selected from a hydrogen atom, halogen, hydrocarbon group, halogenated hydrocarbon group, oxygen-containing hydrocarbon group, silicon-containing hydrocarbon group, cyanide-containing

hydrocarbon group or nitrogen-containing hydrocarbon group.

Optionally, $R_5$ and $R_6$ are independently selected from hydrogen atom, fluorine atom, methyl, ethyl, propyl, isopropyl, butyl, tert-butyl, alkylene, alkynyl, phenyl, trimethylsilyl, cyano, trifluoromethyl, tetrafluoroethyl, naphthyl, tetrafluorophenyl, pyrrolyl or pyridyl.

Optionally, a mass percentage of the compound represented by Structural Formula 1 in the non-aqueous electrolyte for a lithium ion battery is 0.05%-10%.

Optionally, the non-aqueous electrolyte further includes one or more of 1,3-propane sultone, 1,4-butane sultone, 1,3-propylene sultone, vinylene carbonate, vinyl ethylene carbonate, fluoroethylene carbonate and ethylene sulfate.

Optionally, the lithium salt is selected from one or more of one or more of $LiPF_6$, $LiBF_4$, LiBOB, $LiPO_2F_2$, LiDFOB, $LiSbF_6$, $LiAsF_6$, $LiN(SO_2CF_3)_2$, $LiN(SO_2C_2F_5)_2$, $LiC(SO_2CF_3)_3$ or $LiN(SO_2F)_2$; and

the non-aqueous organic solvent is a mixture of cyclic carbonate and chain carbonate, the cyclic carbonate is selected from one or more of ethylene carbonate, propylene carbonate or butylene carbonate, and the chain carbonate is selected from one or more of dimethyl carbonate, diethyl carbonate, ethyl methyl carbonate or methyl propyl carbonate.

In another aspect, the embodiment of the present application provides a lithium ion battery, including a positive electrode, a negative electrode, a separator arranged between the positive electrode and the negative electrode, and an electrolyte, wherein the electrolyte is selected from the above-mentioned non-aqueous electrolyte for a lithium ion battery.

Optionally, the positive electrode includes a positive electrode active material, the positive electrode active material includes one or more of $LiCoO_2$, $LiNiO_2$, $LiMn_2O_4$, $LiCo_{1-y}M_yO_2$, $LiNi_{1-y}M_yO_2$, $LiMn_{2-y}M_yO_4$, $LiCo_xM_{(1-x)}O_2$ or $LiNi_xCo_yMn_zM_{1-x-y-z}O_2$; wherein M is selected from one or more of Al, Sr, Mg, Ti, Ca, Zr, Zn, Si, Cu, V or Fe, and $0 \le x \le 1$, $0 \le y \le 1$, $0 \le z \le 1$, $x+y+z \le 1$.

The non-aqueous electrolyte provided by the present application includes the compound represented by Structural Formula 1, which not only has the characteristic of sulfonate additives to improve high-temperature storage performance of battery, but also has the characteristic of sulfate additives to improve high-temperature cycle performance of battery. In the first charging process, the sulfonate structure in Structural Formula 1 can take precedence over solvent molecules in the reduction and decomposition reaction at positive electrode, and the reaction product forms a passivation film on the surface of the positive electrode. The passivation film can inhibit further decomposition of solvent molecules in the electrolyte. The strong coordination between atom O of sulfonate structure and $Li^+$ leads to the disconnection of the five-membered ring of 1,3-PS (1,3-propane sultone) of Structural Formula 1, and $Li^+$ obtains an electron to form an anionic free radical. Because anionic free radicals are very active and highly reactive, lithium sulfonate is further generated, thus forming a passivation film on the surface of the positive electrode, and the functional group X is further crosslinked to make the passivation film more dense and stable, which can effectively improve the electrochemical performance of the electrode, the storage performance and self-discharge performance of the battery.

## Detailed description of preferred embodiments

In order to make the beneficial effects, technical solutions and technical problems solved by the present application clearer, the present application will be further illustrated in detail with reference to the drawings and embodiments. It should be understood that the specific embodiments described here are merely for illustrating the present application, not for limiting the present application.

The non-aqueous electrolyte for a lithium ion battery provided by an embodiment of the present application includes a non-aqueous organic solvent, a lithium salt, and a spiro compound represented by Structural Formula 1,

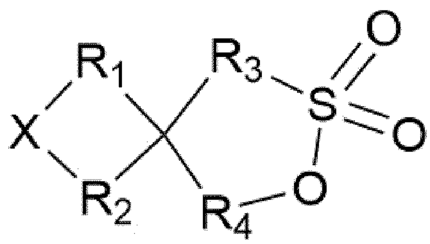

(Structural Formula 1)

wherein, X represents an oxygen atom, a carboxylate ester, a sulfite ester, a sulfate ester, a carbonate ester, a substituted or unsubstituted phosphate ester, and a substituted or unsubstituted borate ester; and

$R_1$, R2, $R_3$ and $R_4$ are each independently selected from substituted or unsubstituted C1-C5 hydrocarbylene group.

The non-aqueous electrolyte for a lithium ion battery provided by the present application includes the compound represented by Structural Formula 1 as an additive, and this asymmetric spirocyclic compound has the characteristic of sulfonate additives to improve high-temperature storage performance of battery, and also has the characteristic of sulfate additives to improve high-temperature cycle performance of battery.

The mechanism is that in the first charging process, the sulfonate structure in Structural Formula 1 can take precedence over solvent molecules in the reduction and decomposition reaction at positive electrode, and the reaction product forms a passivation film on the surface of the positive electrode. The passivation film can inhibit further decomposition of solvent molecules in the electrolyte. The strong coordination between atom O of sulfonate structure and Li+ leads to the disconnection of the five-membered ring of 1,3-PS (1,3-propane sultone) of Structural Formula 1, and Li+ obtains an electron to form an anionic free radical. Because anionic free radicals are very active and highly reactive, lithium sulfonate is further generated, and a passive film is deposited on the surface of the positive electrode. In the first charging process, the sulfate structure of Structural Formula 1 can improve the composition of SEI film, slow down the exothermic decomposition of SEI film at high temperature, and improve the high-temperature cycle performance of the battery cooperatively.

The functional group X may be selected from oxygen atoms, carboxylate group, sulfite group, sulfate group, carbonate group, substituted or unsubstituted phosphate group, and substituted or unsubstituted borate group. These groups all have crosslinking activity, and further cross-link with solvent molecules in the electrolyte, which makes the passive film coated on the surface of the positive electrode more compact and stable, and can effectively improve the electrochemical performance of the electrode, the storage performance and self-discharge performance of the battery.

In one embodiment, the spiro compound represented by Structural Formula 1 has an asymmetric structure, and the asymmetric structure represented by Structural Formula 1 includes a five-membered ring structure similar to 1,3-PS and a multi-membered ring structure of cyclic lactone, which can improve the high-temperature cycle performance of battery cooperatively.

In one embodiment, at least one of $R_1$, $R_2$, $R_3$ and $R_4$ is a substituted or unsubstituted C1-C5 alkylene group; a substituent of C1-C5 alkylene group includes one or more of a halogen-substituted or unsubstituted C1-C20 alkyl group, a halogen-substituted or unsubstituted C2-C20 alkenyl group, a halogen-substituted or unsubstituted C2-C20 alkynyl group, a halogen-substituted or unsubstituted C3-C20 cycloalkenyl group, a halogen-substituted or unsubstituted C3-C20 heterocyclic group, a halogen-substituted or unsubstituted C6-C40 aryl group, a halogen-substituted or unsubstituted C2-C40 heteroaryl group or a polar functional group with at least one heteroatom.

It should be noted that when $R_1$, $R_2$, $R_3$ and $R_4$ are independently select from alkylene group, it is preferable that the number of carbon atoms be controlled to 5 or less (including 5). When the number of carbon atoms is controlled below 5, the high-pemperature performance can be guaranteed as well. However, when the number of carbon atoms in the alkyl group is more than 6, the more carbon atoms there are, the more the relative density of the alkyl group will gradually increase, which will adversely affect the high-temperature performance and inflation inhibition of battery.

According to the present application, when the number of carbon atoms is less than 5 or more than 6, the effects of improving the SEI film and improving the high-temperature cycle performance of battery can be achieved. Preferably, the number of carbon atoms of alkylene groups each independently selected by $R_1$, $R_2$, $R_3$ and $R_4$ is controlled to be less than 5.

In one embodiment, the substituent of C1-C5 alkylene group includes one or more of halogen, methyl, ethyl, propyl, isopropyl, butyl, tert-butyl, alkylene, alkynyl, phenyl, trimethylsilyl, cyano, trifluoromethyl, tetrafluoroethyl, naphthyl, tetrafluorophenyl, pyrrolyl or pyridyl.

In one embodiment, the spiro compound represented by Structural Formula 1 include at least one of Formulas 1 to 7,

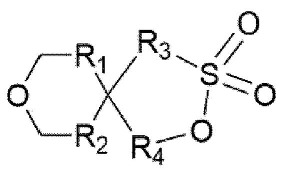

Formula 1

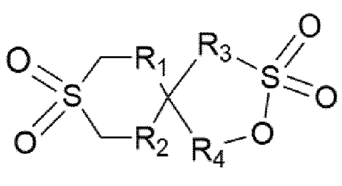

Formula 2

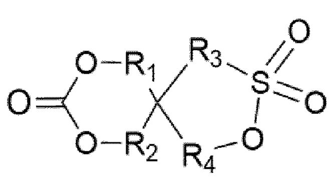

Formula 3

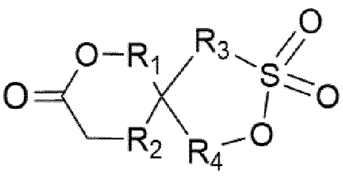

Formula 4

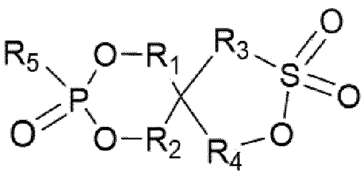

Formula 5

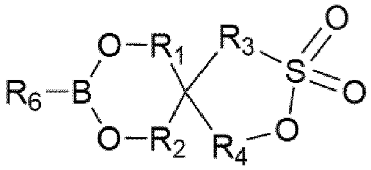

Formula 6

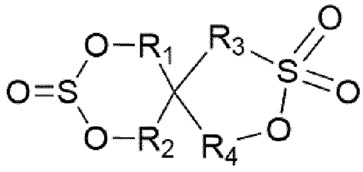

Formula 7

wherein $R_5$ and $R_6$ are each independently selected from a hydrogen atom, halogen, hydrocarbon group, halogenated hydrocarbon group, oxygen-containing hydrocarbon group, silicon-containing hydrocarbon group, cyanide-containing hydrocarbon group or nitrogen-containing hydrocarbon group.

In one embodiment, $R_5$ and $R_6$ are independently selected from hydrogen atom, fluorine atom, methyl, ethyl, propyl, isopropyl, butyl, tert-butyl, alkylene, alkynyl, phenyl, trimethylsilyl, cyano, trifluoromethyl, tetrafluoroethyl, naphthyl, tetrafluorophenyl, pyrrolyl or pyridyl.

In one embodiment, the compound represented by Structural Formula 1 may be:

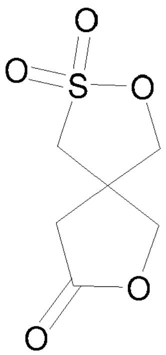

Compound 1

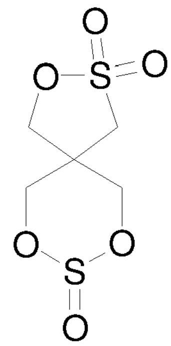

Compound 2

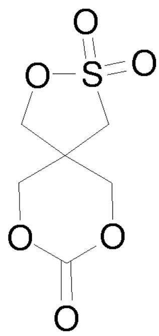

Compound 3

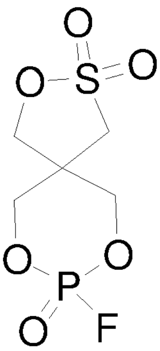

Compound 4

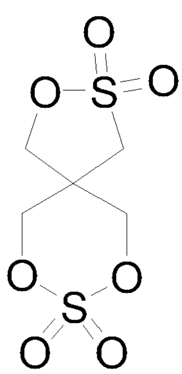

Compound 5

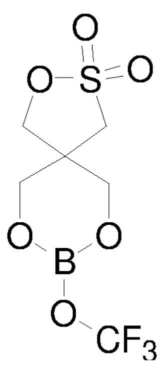

Compound 6

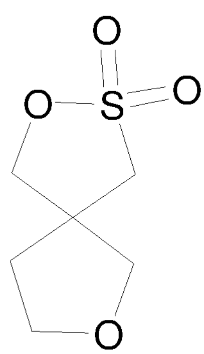

Compound 7

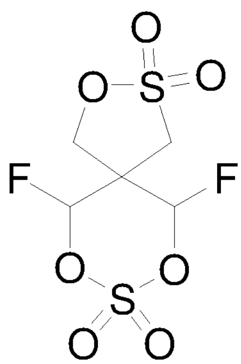

Compound 8.

It should be noted that the above compounds are part of the claimed subject matter of the present application, but not limited to this, and should not be considered as limitations to the present application.

The addition amount of the compound represented by Structural Formula 1 in the present application is not intended to limit the present application. However, controlling the content of the compound represented by Structural Formula 1 in non-aqueous electrolyte has a favorable influence on further optimization of high-temperature performance.

In one embodiment, based on the total mass of the non-aqueous electrolyte for a lithium ion battery being 100%, a mass percentage of the compound represented by Structural Formula 1 in the non-aqueous electrolyte for a lithium ion battery is 0.05%-10%. Specifically, the mass percentage of the compound represented by Structural Formula 1 may be 0.05%, 0.08%, 0.1%, 0.5%, 0.8%, 1%, 1.2%, 1.5%, 1.8%, 2%, 2.2%, 2.5%, 2.8%, 3%, 3.2%, 3.5%, 3.8%, 4%, 4.5%, 5%, 5.5%, 6%, 6.5%, 7%, 7.5%, 7.8%, 8%, 8.5%, 9%, 9.5% and 10%.

Within the above range, the compound represented by Structural Formula 1 may obviously improve the high-temperature storage performance and high-temperature cycle performance of battery, and the battery will not swell as well. When the mass percentage of the compound represented by Structural Formula 1 is less than 0.05%, the content of the compound represented by Structural Formula 1 in the electrolyte is too low to form a complete passivation film on the surface of the positive electrode, thus it is unlikely to visibly improve high-temperature performance of the non-aqueous electrolyte, and the internal resistance of battery would not be significantly reduced. However, when the mass percentage of the compound represented by Structural Formula 1 exceeds 10.0%, an excessively thick passivation film would likely to be formed on the surface of the positive electrode, which increases the internal resistance of battery, and the battery capacity retention rate is obviously deteriorated.

In an embodiment, additives in the non-aqueous electrolyte further include 1,3-propane sultone (1,3-PS), 1,4-butane sultone (BS), 1,3-propylene sultone (PST), vinylene carbonate (VC), vinyl ethylene carbonate (VEC), fluoroethylene carbonate (FEC) and ethylene sulfate (DTD). These additives may be used in combination with the compound represented by Structural Formula 1 to form a more stable SEI film on the surface of graphite negative electrode, thus significantly improving the cycle performance of lithium-ion batteries, and obtaining more excellent effects compared with those adding compound represented by Structural Formula 1 alone.

Based on the total mass of the non-aqueous electrolyte for a lithium ion battery being 100%, the mass percent of the additive in the non-aqueous electrolyte for a lithium ion battery is 0.05-10%. Preferably, the mass percentage of addictive in the non-aqueous electrolyte for a lithium ion battery is 0.2-5%, and more preferably, the mass percentage of addictive in the non-aqueous electrolyte for a lithium ion battery is 0.5-2%. Specifically, the mass percentage of the additive may be 0.05%, 0.08%, 0.1%, 0.5%, 0.8%, 1%, 1.2%, 1.5%, 1.8%, 2%, 2.2%, 2.5%, 2.8%, 3%, 3.2%, 3.5%, 3.8%, 4%, 4.5%, 5%, 5.5%, 6%, 6.5%, 7%, 7.5%, 7.8%, 8%, 8.5%, 9%, 9.5% and 10%.

In one embodiment, the non-aqueous organic solvent is a mixture of cyclic carbonate and chain carbonate. The cyclic carbonate includes one or more of ethylene carbonate, propylene carbonate and butylene carbonate. The chain carbonate includes one or more of dimethyl carbonate, diethyl carbonate, ethyl methyl carbonate and methyl propyl carbonate.

There is no specific limitation on lithium salt in the solution of the present application, and various existing substances may be used.

In an embodiment, the lithium salt is selected from one or more of one or more of $LiPF_6$, $LiBF_4$, LiBOB, $LiPO_2F_2$, LiDFOB, $LiSbF_6$, $LiAsF_6$, $LiN(SO_2CF_3)_2$, $LiN(SO_2C_2F_5)_2$, $LiC(SO_2CF_3)_3$ or $LiN(SO_2F)_2$. Preferably, the lithium salt is $LiPF_6$ or a mixture of $LiPF_6$ with other lithium salts.

Based on the total mass of the non-aqueous electrolyte for a lithium ion battery being 100%, the mass percentage of the lithium salt in the non-aqueous electrolyte for a lithium ion battery is 0.1-15%

Another embodiment of the present application discloses a lithium ion battery, which includes a positive electrode, a negative electrode, a separator and an electrolyte, wherein the electrolyte is the non-aqueous electrolyte for a lithium ion battery described above.

As in the prior art, non-aqueous organic solvent and lithium salt are both contained in the non-aqueous electrolyte for a lithium ion batteries. For the solution of the present application, there are no special limitations on the types or contents of the solvent and lithium salt, and various existing substances may be used.

In an embodiment, the positive electrode includes a positive electrode active material, the positive electrode active material includes one or more of $LiCoO_2$, $LiNiO_2$, $LiMn_2O_4$, $LiCo_{1-y}M_yO_2$, $LiNi_{1-y}M_yO_2$, $LiMn_{2-y}M_yO_4$, $LiCo_xM_{(1-x)}O_2$ or $LiNi_xCo_yMn_zM_{1-x-y-z}O_2$; wherein M is selected from one or more of Al, Sr, Mg, Ti, Ca, Zr, Zn, Si, Cu, V or Fe, and $0 \le x \le 1$, $0 \le y \le 1$, $0 \le z \le 1$, $x+y+z \le 1$.

The positive electrode further includes a positive electrode current collector for extracting current, and the positive electrode active material covers the positive electrode current collector.

The negative electrode includes a negative electrode active material, and the negative electrode active material includes a graphite, a conductive agent, SBR (styrene butadiene rubber), CMC (sodium carboxymethyl cellulose) and NMP (N- methylpyrrolidone).

The negative electrode further includes a negative electrode current collector for extracting current, and the

negative electrode active material covers the negative electrode current collector.

In some embodiments, there is a separator between the battery positive electrode and battery negative electrode, and the separator is a conventional diaphragm in the field of lithium ion batteries, which will not be described in detail here.

The present application will be further illustrated with embodiments.

Embodiment 1

This embodiment is used to illustrate the non-aqueous electrolyte for a lithium ion battery, lithium ion battery and its preparation method, which are disclosed in the present application. The preparation process includes the following steps.

1) Preparation of non-aqueous electrolyte

Ethylene carbonate (EC), diethyl carbonate (DEC) and ethyl methyl carbonate (EMC) were mixed according to the mass ratio of EC: DEC: EMC = 1: 1: 1, and then lithium hexafluorophosphate (LiPF6) was added until the molar concentration was 1 mol/L. Based on the total mass of the non-aqueous electrolyte being 100%, 1.0% of Compound 1 was added.

2) Preparation of positive electrode plate

According to the mass ratio of 93:4:3, the positive electrode active material $LiNi_{0.5}Co_{0.2}Mn_{0.3}O_2$, conductive carbon black Super-P and binder polyvinylidene fluoride (PVDF) were mixed, and then they were dispersed in N-methyl -2-pyrrolidone (NMP), and uniformly mixed to obtain a positive electrode slurry for lithium ion batteries. Both sides of the aluminum foil of the positive electrode current collector were evenly coated with the positive electrode slurry. After vacuum dried, calendered and trimmed, it was cut into pieces and divided into strips. And the aluminum lead-out wire was welded by an ultrasonic welder to obtain a positive electrode plate with a thickness of 120-150μm.

3) Preparation of negative electrode plate

According to the mass ratio of 94:1:2.5:2.5, the negative electrode active material of artificial graphite, conductive carbon black Super-P, binder styrene-butadiene rubber (SBR) and thickener carboxymethyl cellulose (CMC) were mixed, and then they were dispersed in deionized water to obtain negative electrode slurry. Both sides of the copper foil were evenly coated with the slurry. After vacuum dried, calendered and trimmed, it was cut into pieces and divided into strips. And the nickel lead-out wire was welded by an ultrasonic welder to obtain a negative electrode plate with a thickness of 120-150μm.

4) Preparation of battery core

A three-layered lithium battery separator with a thickness of 20μm was placed between the prepared positive electrode and negative electrode, and the sandwich structure consisting of the positive electrode, negative electrode and separator was wound, then the wound body was pressed flat and put into an aluminum foil packaging bag, then baked in vacuum at 75°C for 48 hours to obtain a battery core to be injected with liquid.

5) Liquid injection and formation of battery core

In a glove box with the dew point controlled below -40°C, the prepared electrolyte was injected into the dried battery core, vacuum-packaged, and left stand for 24 hours. Then, the first charge was normalized according to the following steps: charged for 180min at 0.05C constant current, then charged to 3.95V at 0.2C constant current, vacuum sealed for a second time, then further charged to 4.2V at 0.2C constant current, and after standing at room temperature for 24 hours, discharged to 3.95 V at 0.2C constant current.

Embodiments 2-15

The battery was prepared according to the preparation process described in Embodiment 1, except that that the types and contents of the compound additives added in the non-aqueous electrolyte during the preparation step were different. See Table 1 for details.

Comparative Example 1

A battery was prepared as described in Embodiment 1, except that: 1.0% of Compound 1 was not added in the non-aqueous electrolyte during the preparation step.

Comparative Examples 2-7

The battery was prepared according to the preparation process described in Embodiment 1, except that that the types and contents of the compound additives added in the non-aqueous electrolyte during the preparation step were different. See Table 1 for details.

Table 1

| Embodiment/Comparative Example | Additive represented by Structural Formula 1 and its content | Other additives and content |
|---|---|---|
| Embodiment 1 | Compound 1:1.0% | - |
| Embodiment 2 | Compound 2:1.0% | - |
| Embodiment 3 | Compound 3:1.0% | - |
| Embodiment 4 | Compound 4:1.0% | - |
| Embodiment 5 | Compound 5:1.0% | - |
| Embodiment 6 | Compound 6:1.0% | - |
| Embodiment 7 | Compound 7:1.0% | - |
| Embodiment 8 | Compound 8:1.0% | - |
| Embodiment 9 | Compound 5: 0.1% | - |
| Embodiment 10 | Compound 5: 2% | - |
| Embodiment 11 | Compound 5: 3% | - |
| Embodiment 12 | Compound 5: 5% | - |
| Embodiment 13 | Compound 5: 1.0% | FEC:1.0% |
| Embodiment 14 | Compound 5: 1.0% | VEC:1.0% |
| Embodiment 15 | Compound 5: 1.0% | VC:1.0% |
| Comparative Example 1 | - | - |
| Comparative Example 2 | - | FEC:1.0% |
| Comparative Example 3 | - | VEC:1.0% |
| Comparative Example 4 | - | VC:1.0% |
| Comparative Example 5 | - | 1,3PS:1.0% |
| Comparative Example 6 | - | 1,3PTS:1.0% |
| Comparative Example 7 | - | DTD:1.0% |

Performance test

In order to verify the performance of the electrode of lithium ion battery of the present application, the lithium ion batteries prepared in the above Embodiments 1-15 and Comparative Examples 1-7 were tested. The specific test method is as follows.

1) High-temperature cycle performance test

At 45°C, the formed battery was charged to 4.2V ($LiNi_{0.5}Co_{0.2}Mn_{0.3}O_2$ / artificial graphite battery) at 1C constant current and constant voltage, then charged at constant voltage until the current dropped to 0.02C, and then discharged

at 1C constant current to 3.0V. After N cycles of charge/discharge, capacity retention rate after the 500th cycle was calculated to evaluate its high-temperature cycle performance.

The calculation formula of the 500th cycle capacity retention rate at 45°C, 1C is as follows:

The 500th cycle capacity retention rate (%) = the 500th cycle discharge capacity / the 1st cycle discharge capacity * 100%.

2) High-temperature storage performance test

The formed lithium-ion battery was charged to 4.2V ($LiNi_{0.5}Co_{0.2}Mn_{0.3}O_2$ / artificial graphite battery) at 1C constant current/constant voltage at room temperature, and the initial discharge capacity and initial battery thickness of battery were measured. After being stored at 60°C for 30 days, the battery was discharged to 3V at 1C, and the retention capacity, recovery capacity and battery thickness after storage were measured.

The calculation formula is as follows:

$$\text{Battery capacity retention rate (\%)} = \text{Retention capacity / Initial capacity} * 100\%;$$

$$\text{Battery capacity recovery rate (\%)} = \text{Recovery capacity / Initial capacity} * 100\%;$$

Thickness expansion rate (%) = (Battery thickness after storage - Initial battery thickness) / Initial battery thickness * 100%.

The performance test results of Embodiments 1-15 and Comparative Examples 1-7 are shown in Table 2.

Table 2

| Embodiment/ Comparative Example | The 500th cycle capacity retention rate (%) at 45°C/1C | After 30 days of storage at 60°C | | |
|---|---|---|---|---|
| | | Capacity retention rate | Capacity recovery rate | Thickness expansion rate |
| Embodiment 1 | 73.9% | 84.6% | 89.9% | 9.8% |
| Embodiment 2 | 82.7% | 85.7% | 90.0% | 7.6% |
| Embodiment 3 | 83.0% | 86.0% | 90.4% | 7.2% |
| Embodiment 4 | 83.8% | 84.8% | 91.7% | 8.5% |
| Embodiment 5 | 83.3% | 84.1% | 91.9% | 7.6% |
| Embodiment 6 | 75.5% | 86.2% | 89.1% | 7.8% |
| Embodiment 7 | 74.8% | 85.0% | 91.3% | 8.5% |
| Embodiment 8 | 75.6% | 84.6% | 90.2% | 6.8% |
| Embodiment 9 | 82.4% | 89.1% | 90.8% | 10.10% |
| Embodiment 10 | 84.4% | 90.5% | 92.1% | 8.6% |
| Embodiment 11 | 83.5% | 89.3% | 91.0% | 9.8% |
| Embodiment 12 | 82.0% | 88.8% | 89.7% | 10.1% |
| Embodiment 13 | 85.5% | 92.2% | 95.1% | 7.8% |
| Embodiment 14 | 84.8% | 92.0% | 94.3% | 8.5% |
| Embodiment 15 | 85.0% | 91.9% | 95.1% | 8.4% |
| Comparative Example 1 | 70.2% | 69.6% | 70.8% | 19.2% |

(continued)

| Embodiment/ Comparative Example | The 500th cycle capacity retention rate (%) at 45°C/1C | After 30 days of storage at 60°C | | |
|---|---|---|---|---|
| | | Capacity retention rate | Capacity recovery rate | Thickness expansion rate |
| Comparative Example 2 | 80.0% | 79.2% | 82.1% | 29% |
| Comparative Example 3 | 82.8% | 79.6% | 83.3% | 12.6% |
| Comparative Example 4 | 83.5% | 78.7% | 83.5% | 26.1% |
| Comparative Example 5 | 71.1% | 81.4% | 88.5% | 8.1% |
| Comparative Example 6 | 70.5% | 82.5% | 89.9% | 7.5% |
| Comparative Example 7 | 75.4% | 81.7% | 88.1% | 9.5% |

As can be seen from the data of Embodiments 1-12 in Table 2, compared with Comparative Examples 1-7, the lithium batteries prepared in Embodiments 1-12 have better high-temperature cycle performance and high-temperature storage performance after adding the compound represented by Structural Formula 1. And according to the data of thickness expansion rate, the gas generation is less after adding the compound represented by Structural Formula 1, thus improving the high-temperature performance of battery, preventing the battery from bulging after being stored at high temperature, and improving the charge-discharge performance and cycle times of battery.

According to the data of Embodiment 5 and Embodiments 9-12 in Table 2, compared with the lithium-ion batteries prepared by adding 1%, 2% or 3% of Compound 5, the high-temperature performance of lithium-ion batteries prepared by adding 0.1% or 5% of Compound 5 in the non-aqueous electrolyte is slightly deteriorated, indicating that too little or too much addition would lead to deterioration of high-temperature performance of lithium-ion battery.

According to the data of Embodiments 13-15 in Table 2, when the compound represented by Structural Formula 1 and FEC (or VEC, VC) are both added to the non-aqueous electrolyte, FEC can cooperate with the compound represented by Structural Formula 1 to form a more stable SEI film on the surface of the graphite negative electrode, which makes the lithium-ion batteries have good high-temperature performance and high-temperature storage performance, thus significantly improving the cycle performance of the lithium-ion batteries.

In Comparative Example 1, no additives are added. By comparing the experimental data, it can be seen that the compactness and stability of SEI film would be affected without any additives, which would affect the charge-discharge performance and cycle performance of lithium-ion batteries, and the high-temperature cycle performance and high-temperature storage performance are not good, the batteries would produce more gas.

Comparing and analyzing the experimental data of Comparative Examples 2~ 4, it can be seen that the lithium-ion batteries of Comparative Examples 2~ 4 have good high-temperature cycle performance, but their high-temperature storage performance is poor, and the gas produced by the batteries is serious, and the batteries swell seriously. Fluoroethylene carbonate (FEC) was added in Comparative Example 2, vinyl ethylene carbonate (VEC) was added in Comparative Example 3, and vinylene carbonate (VC) was added in Comparative Example 4. As additives, these substances could take precedence over solvent molecules to undergo reductive decomposition reaction on the negative electrode surface to generate polyalkoxylithium carbonate compounds, and a passive film was formed on the negative electrode surface to prevent the electrolyte from further decomposing on the electrode surface, thus improving the cycle performance of battery. However, after adding FEC, VEC or VC, the battery is likely to generate gas during high temperature storage, which leads to serious swelling of battery.

Comparing and analyzing the experimental data of Comparative Examples 5-7, it can be seen that the lithium-ion batteries in Comparative Examples 5-7 have good high-temperature storage performance, less gas generation and less battery swelling, however their high-temperature cycle performance is poor. This is because when 1,3-propane sultone (1,3-PS) added in Comparative Example 5, 1,3-propylene sultone (PST) added in Comparative Example 6, and ethylene sulfate (DTD) added in Comparative Example 7; as additives, they could suppress the decrease of initial capacity of battery, and increase initial discharge capacity. The battery expansion after high-temperature storage may be reduced, and the charge-discharge performance and cycle times of battery may be improved. However, the stability of the SEI film formed on the graphite negative electrode interface is poor. With the increase of cycle times, the resistance of the electrode interface would gradually increase, and the phenomenon of electronic polarization of battery would become more and more serious, which leads to the rapid decrease of reversible capacity of electrode and the degradation of high-temperature cycle performance of battery.

Through the comparison among the embodiments and comparative examples, it is concluded that the high-temperature cycle performance and high-temperature storage performance of battery are excellent when the compound

represented by Structural Formula 1 is added to the electrolyte, or when the compound represented by Structural Formula 1 and other additives are added at the same time.

The above descriptions are only preferred embodiments of the present application and are not intended to limit the present application.

## Claims

1. A non-aqueous electrolyte for a lithium ion battery, comprising a non-aqueous organic solvent, a lithium salt, and a spiro compound represented by Structural Formula 1,

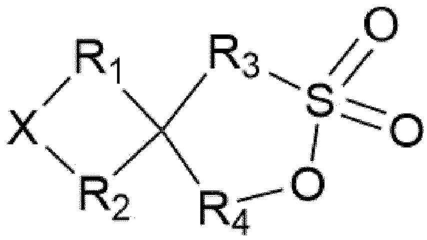

(Structural Formula 1)

wherein, X represents an oxygen atom, a carboxylate ester, a sulfite ester, a sulfate ester, a carbonate ester, a substituted or unsubstituted phosphate ester, and a substituted or unsubstituted borate ester; and
$R_1$, $R_2$, $R_3$ and $R_4$ are each independently selected from substituted or unsubstituted C1-C5 hydrocarbylene group.

2. The non-aqueous electrolyte for a lithium ion battery of claim 1, wherein at least one of $R_1$, $R_2$, $R_3$ and $R_4$ is a substituted or unsubstituted C1-C5 alkylene group; a substituent of C1-C5 alkylene group comprises one or more of a halogen-substituted or unsubstituted C1-C20 alkyl group, a halogen-substituted or unsubstituted C2-C20 alkenyl group, a halogen-substituted or unsubstituted C2-C20 alkynyl group, a halogen-substituted or unsubstituted C3-C20 cycloalkenyl group, a halogen-substituted or unsubstituted C3-C20 heterocyclic group, a halogen-substituted or unsubstituted C6-C40 aryl group, a halogen-substituted or unsubstituted C2-C40 heteroaryl group or a polar functional group with at least one heteroatom.

3. The non-aqueous electrolyte for a lithium ion battery of claim 2, wherein the substituent of C1-C5 alkylene group is selected from one or more of halogen, methyl, ethyl, propyl, isopropyl, butyl, tert-butyl, alkylene, alkynyl, phenyl, trimethylsilyl, cyano, trifluoromethyl, tetrafluoroethyl, naphthyl, tetrafluorophenyl, pyrrolyl or pyridyl.

4. The non-aqueous electrolyte for a lithium ion battery of claim 1, wherein the spiro compound represented by Structural Formula 1 comprise at least one of Formulas 1 to 7,

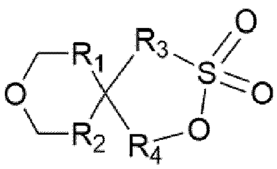

Formula 1

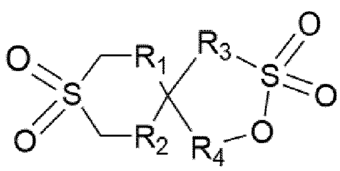

Formula 2

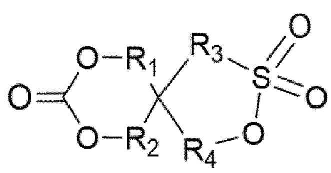

Formula 3

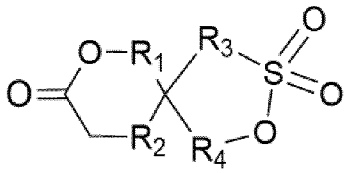 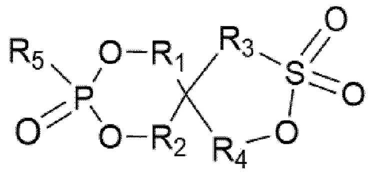

Formula 4 Formula 5

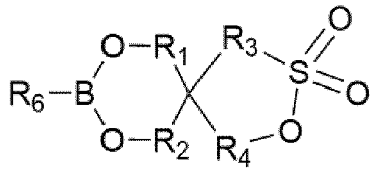 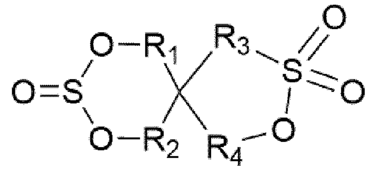

Formula 6 Formula 7

wherein $R_5$ and $R_6$ are each independently selected from a hydrogen atom, halogen, hydrocarbon group, halogenated hydrocarbon group, oxygen-containing hydrocarbon group, silicon-containing hydrocarbon group, cyanide-containing hydrocarbon group or nitrogen-containing hydrocarbon group.

**5.** The non-aqueous electrolyte for a lithium ion battery of claim 4, wherein $R_5$ and $R_6$ are independently selected from hydrogen atom, fluorine atom, methyl, ethyl, propyl, isopropyl, butyl, tert-butyl, alkylene, alkynyl, phenyl, trimethylsilyl, cyano, trifluoromethyl, tetrafluoroethyl, naphthyl, tetrafluorophenyl, pyrrolyl or pyridyl.

**6.** The non-aqueous electrolyte for a lithium ion battery of claim 1, wherein a mass percentage of the compound represented by Structural Formula 1 in the non-aqueous electrolyte for a lithium ion battery is 0.05%-10%.

**7.** The non-aqueous electrolyte for a lithium ion battery of claim 1, wherein the non-aqueous electrolyte further comprises one or more of 1,3-propane sultone, 1,4-butane sultone, 1,3-propylene sultone, vinylene carbonate, vinyl ethylene carbonate, fluoroethylene carbonate and ethylene sulfate.

**8.** The non-aqueous electrolyte for a lithium ion battery of claim 1, wherein the lithium salt is selected from one or more of $LiPF_6$, $LiBF_4$, LiBOB, $LiPO_2F_2$, LiDFOB, $LiSbF_6$, $LiAsF_6$, $LiN(SO_2CF_3)_2$, $LiN(SO_2C_2F_5)_2$, $LiC(SO_2CF_3)_3$ or $LiN(SO_2F)_2$; and
the non-aqueous organic solvent is a mixture of cyclic carbonate and chain carbonate, the cyclic carbonate is selected from one or more of ethylene carbonate, propylene carbonate or butylene carbonate, and the chain carbonate is selected from one or more of dimethyl carbonate, diethyl carbonate, ethyl methyl carbonate or methyl propyl carbonate.

**9.** A lithium ion battery, comprising a positive electrode, a negative electrode, a separator arranged between the positive electrode and the negative electrode, and an electrolyte, wherein the electrolyte is the non-aqueous electrolyte for a lithium ion battery of any one of claims 1-8.

**10.** The lithium ion battery of claim 9, wherein the positive electrode comprises a positive electrode active material, the positive electrode active material comprises one or more of $LiCoO_2$, $LiNiO_2$, $LiMn_2O_4$, $LiCo_{1-y}M_yO_2$, $LiNi_{1-y}M_yO_2$, $LiMn_{2-y}M_yO_4$, $LiCo_xM_{(1-x)}O_2$ or $LiNi_xCo_yMn_zM_{1-x-y-z}O_2$; wherein M is selected from one or more of Al, Sr, Mg, Ti, Ca, Zr, Zn, Si, Cu, V or Fe, and $0 \le x \le 1$. $0 \le y \le 1$, $0 \le z \le 1$, $x+y+z \le 1$.

**11.** The non-aqueous electrolyte for a lithium ion battery of claim 1, wherein the spiro compound represented by Structural Formula 1 has an asymmetric structure.

**12.** The non-aqueous electrolyte for a lithium ion battery of claim 1, wherein the compound represented by Structural Formula 1 is:

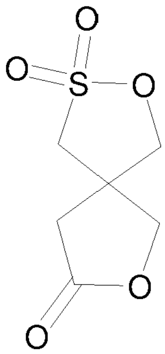

Compound 1

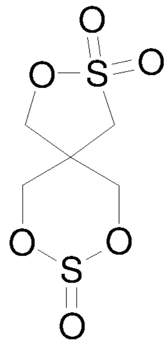

Compound 2

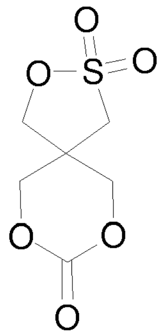

Compound 3

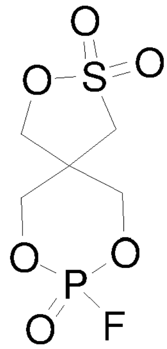

Compound 4

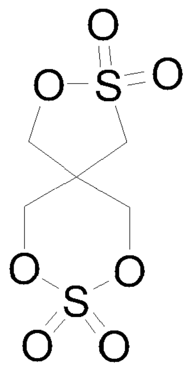

Compound 5

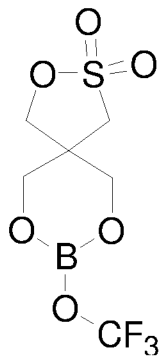

Compound 6

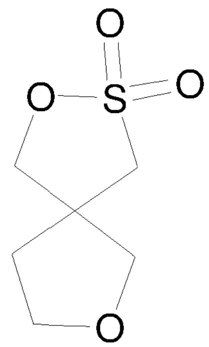

Compound 7

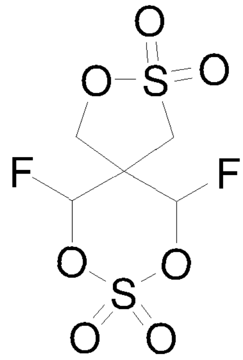

Compound 8

**Patentansprüche**

1. Ein nicht wässriger Elektrolyt für eine Lithium-Ionen-Batterie, der ein nicht wässriges organische Lösemittel, ein Lithiumsalz und eine Spiroverbindung umfassen, wie in der Strukturformel 1 dargestellt ist.

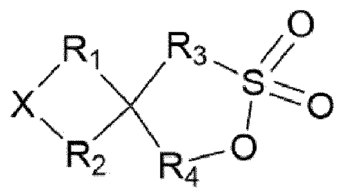

(Strukturformel 1)

Bei der X ein Sauerstoffatom, ein Carboxylatester, ein Sulfitester, ein Sulfatester, ein Carbonatester, ein subsistuiertes oder nichtsubsistuiertes Phosphatester und ein substituiertes oder nicht substituiertes Boratester darstellt, und
$R_1$, $R_2$, $R_3$ und $R_4$ jedes unabhängig von substituierten oder nichtsubstituierten C1-C5 Hydrocarbylengruppen gewählt wird.

2. Der nicht wässrige Elektrolyt für eine Lithium-Ionen-Batterie gemäss Anspruch 1, bei dem mindestens einer von $R_1$, $R_2$, $R_3$ und $R_4$ eine substituierte oder nichtsubstituiertes C1-C5 Alkylengruppe ist, ein Substituent der C1-C5 Alkylengruppe eine oder mehrere halogensubstituierte oder nicht halogensubstituierte C1-C20 Alkylgruppe, eine halogensubstituierte oder nicht halogensubstituierte C2-C20 Alkenylgruppe, halogensubstituierte oder nicht halogensubstituierte C2-C20 Alkenylgruppe, eine halogensubstituierte oder nicht halogensubstituierte C2-C20 Alkynylgruppe, eine halogensubstituierte oder nicht halogensubstituierte C3-C20 Cycloalkenylgruppe, eine halogensubstituierte oder nicht halogensubstituierte C3-C20 heterozyklische Gruppe, eine halogensubstituierte oder nicht halogensubstituierte C6-C40 Arylgruppe, eine halogensubstituierte oder nicht halogensubstituierte C2-C40 Heteroarylgruppe oder eine polarfunktionale Gruppe mit mindestens einem Heteroatom.

**3.** Der nicht wässrige Elektrolyt für eine Lithium-Ionen-Batterie gemäss Anspruch 2, bei dem das Substituent der C1-C5 Alkylengruppe ausgewählt wird aus einem oder mehreren folgender Substituenten: Halogen, Methyl, Ethyl, Propyl, Isopropyl, Butyl, tert-Buthyl, Alkylen, Alkynyl, Phenyl, Trimethylsilyl, Cyan, Trifluoromethyl, Tetrafluoroethyl, Naphtyl, Tetrafluorophenyl, Pyrrolyl oder Pyridyl.

**4.** Der nicht wässrige Elektrolyt für eine Lithium-Ionen-Batterie gemäss Anspruch 1, bei dem die Spiroverbindung gemäss der Strukturformel 1 mindestens eine der Formeln 1 bis 7 umfasst.

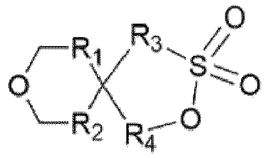
Formel 1

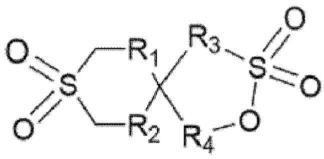
Formel 2

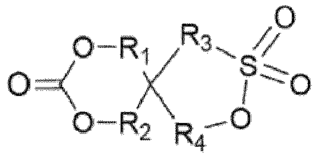
Formel 3

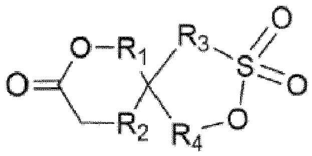
Formel 4

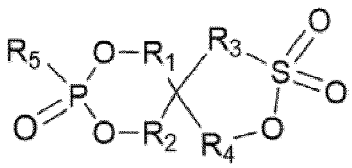
Formel 5

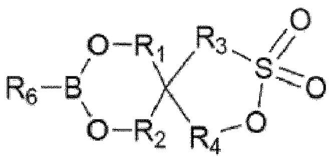
Formel 6

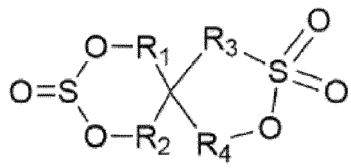
Formel 7

Wobei $R_5$ und $R_6$ jeder unabhängig ausgewählt wird aus Folgendem: Ein Wasserstoffatom, Halogen, eine Kohlenwasserstoffgruppe, eine halogenierte Kohlenwasserstoffgruppe, eine sauerstoffhaltige Kohlenwasserstoffgruppe, eine siliziumhaltige Kohlenwasserstoffgruppe, eine cyanidhaltige Kohlenwasserstoffgruppe oder eine stickstoffhaltige Kohlenwasserstoffgruppe.

**5.** Der nicht wässrige Elektrolyt für eine Lithium-Ionen-Batterie gemäss Anspruch 4, bei dem $R_5$ und $R_6$ unabhängig ausgewählt werden unter einem Wasserstoffatom, Fluorinatom, Methyl, Ethyl, Propyl, Butyl, tert-Butyl, Alkylen, Alkynyl, Phenyl, Trimethylsilyl, Cyan, Trifluoromethyl, Tetrafluoroethyl, Naphthyl, Tetrafluorophenyl, Pyrrolyl oder Pyridyl.

**6.** Der nicht wässrige Elektrolyt für eine Lithium-Ionen-Batterie gemäss Anspruch 1, bei dem ein Masseprozent der durch die Strukturformel 1 dargestellten Verbindung im nicht wässrigen Elektrolyt für eine Lithium-Ionen-Batterie 0,05%-10% beträgt.

**7.** Der nicht wässrige Elektrolyt für eine Lithium-Ionen-Batterie gemäss Anspruch 1, wobei das nicht wässrige Elektrolyt weiter eine oder mehrere folgender Verbindungen umfasst: 1,3-Propan-Sulton, 1,4-Butan-Sulton, 1,3-Propylen-Sulton, Vinlencarbonat, Vinyl-Ethylen-Carbonat, Fluoroethylen-Carbonat und Ethylensulfat.

**8.** Der nicht wässrige Elektrolyt für eine Lithium-Ionen-Batterie gemäss Anspruch 1, bei dem das Lithiumsalz ausgewählt

wird aus einer oder mehreren Verbindungen: $LiPF_6$, $LiBF_4$, LiBOB, $LiPO_2F_2$, LiDFOB, $LiSbF_6$, $LiAsF_6$, $LiN(SO_2CF_3)_2$, $LiN(SO_2C_2F5)_2$, $LiC(SO_2CF_3)_3$ oder $LiN(SO_2F)_2$; und das nicht wässrige, organische Lösemittel eine Mischung von zyklischem Carbonat und Ketten-Carbonat ist, das zyklische Carbonat ausgewählt wird aus einem oder mehreren folgender Verbindungen: Ethylen-Carbonat, Propylen-Carbonat oder Butylen-Carbonat und das Kettencarbonat aus folgenden Verbindungen: Dimethyl-Carbonat, Diethyl-Carbonat, Ethyl-Methyl-Carbonat oder Methyl-Propyl-Carbonat.

**9.** Eine Lithium-Ionen-Batterie, die eine positive Elektrode, eine negative Elektrode, ein zwischen der positiven Elektrode und negativen Elektrode angeordnetes Trennelement und einen Elektrolyt umfasst wobei der Elektrolyt ein nicht wässriger Elektrolyt für eine Lithiumionbatterie gemäss irgendeinem der Ansprüche 1-8 ist.

**10.** Die Lithium-Ionen-Batterie gemäss Anspruch 9, bei der die positive Elektrode ein aktives Material der positiven Elektroden umfasst, das aktive Material für positive Elektroden eine oder mehrere folgender Verbindungen umfasst: $LiCoO_2$, $LiNiO_2$, $LiMn_2O_4$, $LiCo_{1-y}M_yO_2$, $LiNi_{1-y}M_yO_2$, $LiMn_{2-y}M_yO_4$, $LiCo_xM_{(1-x)}O_2$ oder $LiNi_xCO_yMn_zM_{l-x-y-z}O_2$; wobei M aus einem oder mehreren folgender Elemente ausgewählt wird: Al, Sr, Mg, Ti, Ca, Zr, Zn, Si, Cu, V oder Fe, und $0\leq x\leq 1$ $0\leq y\leq 1$, $0\leq z\leq 1$, $x+y+z\leq 1$.

**11.** Das nicht wässrige Elektrolyt für eine Lithium-Ionen-Batterie gemäss Anspruch 1, bei dem die durch die Strukturformel 1 dargestellte Spiroverbindung eine asymmetrische Struktur aufweist.

**12.** Das nicht wässrige Elektrolyt für eine Lithium-Ionen-Batterie gemäss Anspruch 1, bei dem die durch die Strukturformel 1 dargestellte Verbindung Folgende ist:

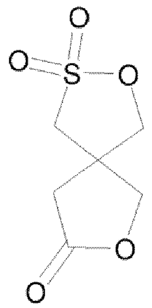

Verbindung 1

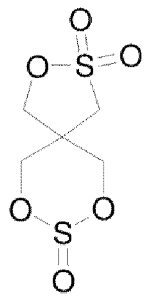

Verbindung 2

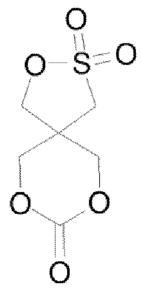

Verbindung 3

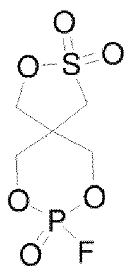

Verbindung 4

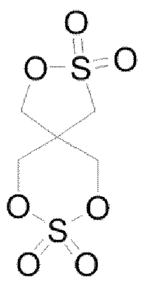

Verbindung 5

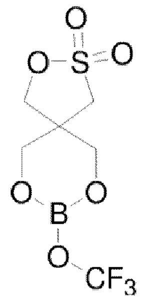

Verbindung 6

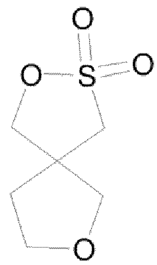

Verbindung 7

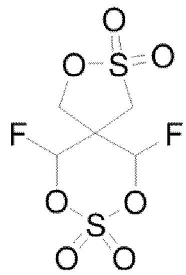

Verbindung 8

## Revendications

**1.** Un électrolyte non aqueux pour une batterie au lithium-ion, comprenant un solvant organique non aqueux, un sel de lithium et un composé spiro représenté par la formule structurelle 1,

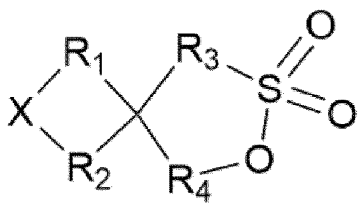

(Formule structurelle 1)

dans laquelle X représente un atome d'oxygène, un ester de carboxylate, un ester de sulfite, un ester de sulfate, un ester de carbonate, un ester de phosphate substitué ou non substitué, et un ester de borate substitué ou non substitué ; et

$R_1$, $R_2$, $R_3$ et $R_4$ sont chacun indépendamment choisi parmi les groupes hydrocarbylène en C1-C5 substitués ou non substitués.

**2.** L'électrolyte non aqueux pour une batterie lithium-ion selon la revendication 1, dans lequel au moins un des $R_1$, $R_2$, $R_3$ et $R_4$ est un groupe alkylène en C1-C5 substitué ou non substitué ; un substituant du groupe alkylène en C1 -C5 comprend un ou plusieurs des groupes suivants : groupe alkyle en C1-C20 substitué ou non substitué par un halogène, groupe alcényle en C2-C20 substitué ou non substitué par un halogène, groupe alcynyle en C2-C20 substitué ou non substitué par un halogène, un groupe cycloalcényle C3-C20 substitué ou non substitué par un halogène, un groupe hétérocyclique C3-C20 substitué ou non substitué par un halogène, un groupe aryle C6-C40 substitué ou non substitué par un halogène, un groupe hétéroaryle C2-C40 substitué ou non substitué par un halogène ou un groupe fonctionnel polaire avec au moins un hétéroatome.

**3.** L'électrolyte non aqueux pour une batterie lithium-ion selon la revendication 2, dans lequel le substituant du groupe alkylène en C1-C5 est choisi parmi un ou plusieurs des groupes suivants : halogène, méthyle, éthyle, propyle, isopropyle, butyle, tert-butyle, alkylène, alcynyle, phényle, triméthylsilyle, cyano, trifluoroinéthyle, tétrafluoroéthyle, naphtyle, tétrafluorophényle, pyrrolyle ou pyridyle.

**4.** L'électrolyte non aqueux pour une batterie au lithium-ion selon la revendication 1, dans lequel le composé spiro représenté par la formule structurelle 1 comprend au moins une des formules 1 à 7,

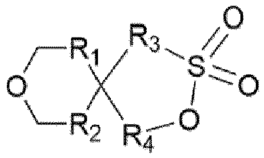

Formule 1

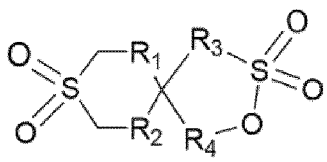

Formule 2

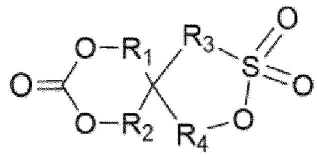

Formule 3

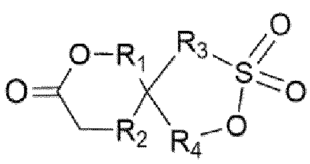

Formule 4

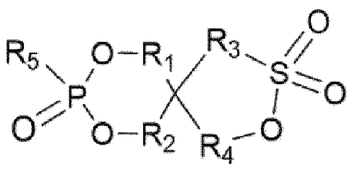

Formule 5

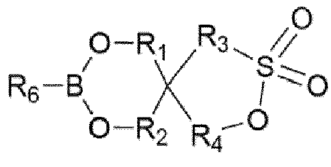

Formule 6

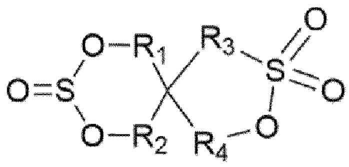

Formule 7

dans lesquelles $R_5$ et $R_6$ sont choisis indépendamment parmi un atome d'hydrogène, un halogène, un groupe d'hydrocarbures, un groupe d'hydrocarbures halogénés, un groupe d'hydrocarbures contenant de l'oxygène, un groupe d'hydrocarbures contenant du silicium, un groupe d'hydrocarbures contenant du cyanure ou un groupe d'hydrocarbures contenant de l'azote.

**5.** L'électrolyte non aqueux pour une batterie au lithium-ion selon la revendication 4, dans lequel $R_5$ et $R_6$ sont indépendamment choisis parmi un atome d'hydrogène, un atome de fluor, un méthyle, un éthyle, un propyle, un isopropyle, un butyle, un tert-butyle, un alkylène, un alcynyle, un phényle, un triméthylsilyle, un cyano, un trifluorométhyle, un tétrafluoroéthyle, un naphtyle, un tétrafluorophényle, un pyirolyle ou un pyridyle.

**6.** L'électrolyte non aqueux pour une batterie au lithium-ion selon la revendication 1, dans lequel un pourcentage massique du composé représenté par la formule structurelle 1 dans l'électrolyte non aqueux pour une batterie au lithium-ion est compris entre 0,05 % et 10 %.

**7.** L'électrolyte non aqueux pour une batterie au lithium-ion selon la revendication 1, dans lequel l'électrolyte non aqueux comprend en outre un ou plusieurs des composés suivants : 1,3-propane sultone, 1,4-butane sultone, 1,3-propylène sultone, carbonate de vinylène, carbonate de vinyle-éthylène, carbonate de fluoroéthylène et sulfate d'éthylène.

**8.** L'électrolyte non aqueux pour une batterie au lithium-ion selon la revendication 1, , dans lequel le sel de lithium est choisi parmi un ou plusieurs des composés suivants : $LiPF_6$, $LiBF_4$, LiBOB, $LiPO_2F_2$, LiDFOB, $LiSbF_6$, $LiAsF_6$, $LiN(SO_2CF_3)_2$, $LiN(SO_2C_2F5)_2$, $LiC(SO_2CF_3)_3$ ou $LiN(SO_2F)_2$ ; et
le solvant organique non aqueux est un mélange de carbonate cyclique et de carbonate en chaîne, le carbonate cyclique étant choisi parmi un ou plusieurs des suivants : carbonate d'éthylène, carbonate de propylène ou carbonate de butylène, et le carbonate en chaîne étant choisi parmi un ou plusieurs des suivants : carbonate de diméthyle, carbonate de diéthyle, carbonate d'éthyle méthyle ou carbonate de méthyle propyle.

**9.** Batterie au lithium-ion, comprenant une électrode positive, une électrode négative, un séparateur disposé entre l'électrode positive et l'électrode négative, et un électrolyte, dans laquelle l'électrolyte est l'électrolyte non aqueux pour une batterie au lithium-ion selon une quelconque des revendications 1-8.

**10.** Une batterie au lithium-ion selon la revendication 9, dans laquelle l'électrode positive comprend un matériau actif d'électrode positive, le matériau actif d'électrode positive comprenant un ou plusieurs des composants suivants : $LiCoO_2$, $LiNiO_2$, $LiMn_2O_4$, $LiCO_{1-y}M_yO_2$, $LiNi_{l-y}M_yO_2$, $LiMn_{2-y}M_yO_4$, $LiCO_xM_{(1-x)}O_2$ ou $LiNi_xCo_yMn_zM_{l-x-y-z}O_2$ ; dans lequel M est choisi parmi un ou plusieurs des éléments suivants : Al, Sr, Mg, Ti, Ca, Zr, Zn, Si, Cu, V ou Fe, et $0 \leq x \leq 1$, $0 \leq y \leq 1$, $0 \leq z \leq 1$, $x+y+z \leq 1$.

**11.** L'électrolyte non aqueux pour batterie au lithium-ion selon la revendication 1, dans lequel le composé spiro représenté par la formule structurale 1 a une structure asymétrique.

**12.** L'électrolyte non aqueux pour une batterie au lithium-ion selon la revendication 1, dans lequel le composé représenté par la formule structurale 1 est :

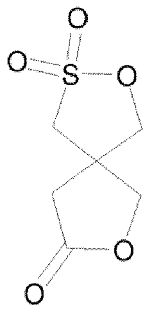

Composé 1

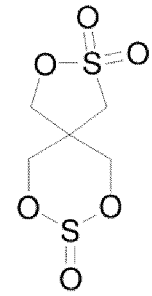

Composé 2

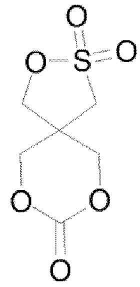

Composé 3

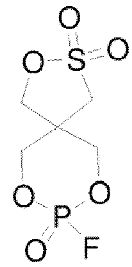

Composé 4

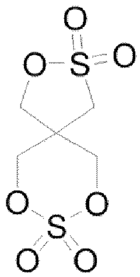

Composé 5

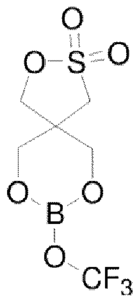

Composé 6

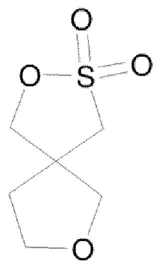

Composé 7

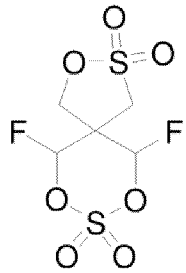

Composé 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000123867 A
- US 2016211550 A1